# EUROPÄISCHE PATENTSCHRIFT

(11) **EP 1 219 964 B1**
(45) Veröffentlichungstag und Bekanntmachung des Hinweises auf die Patenterteilung: **05.04.2006**
(21) Anmeldenummer: 01128717.4
(22) Anmeldetag: 03.12.2001
(51) Int. Cl.: G01N 33/543

(54) **Nachweisverfahren, in welchem der High-Dose-Hook-Effekt vermieden, verringert oder nachgewiesen wird**
Assay method, in which the High-Dose-Hook-Effect is avoided, reduced or detected
Méthode de détection, dans laquelle le "High-Dose-Hook Effect" est évité, réduit ou détecté

(30) Priorität: 22.12.2000 DE 10064827
(43) Veröffentlichungstag der Anmeldung: 03.07.2002
(73) Patentinhaber: Dade Behring Marburg GmbH, 35001 Marburg (DE)
(72) Erfinder: Schelp, Carsten, Dr., Hockessin, DE 19707 (US); Pauly, Hans-Erwin, Dr., 35232 Dautphetal (DE)

(56) Entgegenhaltungen:
- EP-A- 0 603 958
- EP-A- 0 781 998
- EP-B- 0 263 401
- US-A- 4 595 661
- ERIKSSON SUSANN ET AL: "Dual-label time-resolved immunofluorometric assay of free and total prostate-specific antigen based on recombinant Fab fragments." CLINICAL CHEMISTRY, Bd. 46, Nr. 5, Mai 2000 (2000-05), Seiten 658-666, XP001026316 ISSN: 0009-9147

## Beschreibung

Die Erfindung betrifft Verfahren zum quantitativen oder qualitativen Nachweis eines Analyten in einer Probe.

Zum Nachweis von Analyten werden verschiedene Testverfahren eingesetzt, z.B. die sogenannten Sandwich-Immunoassays. Bei einem solchen Immunoassay binden an zwei Epitope des nachzuweisenden Analyten zwei Antikörper, so daß sich ein "Sandwichkomplex" ausbildet.

Bei einem heterogenen Sandwich-Immunoassay ist der eine Antikörper an eine feste Phase (zB. eine Mikrotitrationsplatte, Magnetpartikel, etc.) gebunden und dient zur Abtrennung des Sandwichkomplexes von der flüssigen Phase, während der andere Antikörper zum Nachweis des Immunkomplexes ein nachweisbares Label (z.B. ein Enzym, ein Fluoreszenz- oder Chemilumineszenzlabel, etc.) trägt Man unterteilt diese Testverfahren welter in sogenannte Einschritt-Sandwich-Immunoassays, bei denen die beiden Antikörperreagenzien simultan mit der Probe inkubiert werden, und in Zweischritt-Sandwich-Immunoassays, bei denen die Probe zunächst mit dem Festphasenreagenz inkubiert wird und nach einem Trenn- und Waschschnitt der festphasengebundene Antikörper-Antigen-Komplex mit dem Nachweisreagenz inkubiert wird.

Ein solcher Sandwich-ImMunoassay zum Nachweis von PSA in einer Probe wurde beispielsweise von Eriksson et al. (Clinical Chemistry, 2000. 46:656-666) beschrieben. Ein an eine Festphase gebundenes Fab-Fragment wird als Fängerrnolekül benutzt und die Detektion des PSA erfolgt mit Hilfe zweier Fab-Fragmente, die eine etwas unterschiedliche Affinität zu PSA aufweisen. Das eine ist mit einem fluoreszierenden Europium-Chelat markiert und erkennt lediglich freies PSA. Das andere Fragment Ist mit einem fluoreszierenden Terbium-Chelat markiert und erkennt sowohl freies, als auch Serpinkomplexiertes PSA. Die Europium und Terbium-Signale werden mittels "Time-resolved fluorescence" gemessen.

Bei einem homogenen Sandwich-Immunoassay, wie z.B. einem nephelometrischen Latextest, werden die Antikörperreagenzien mit der Probe zusammen inkubiert und gemessen, wobei an keiner Stelle des Verfahrens ein Trenn- oder Waschschritt durchgeführt wird. Mit anderen. Worten ausgedrückt Es folgt keine Trennung des Antikörper-gebundenen Analyten vom freien Amalyten.

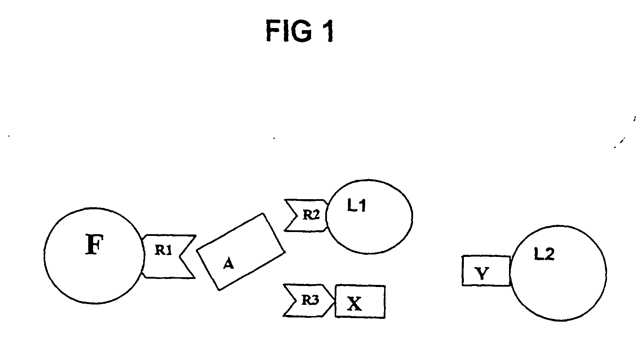

Der Elnschritt-Immunoassay hat wie der homogene Sandwicti-Imrnunoassay den Vorteil einer schnelleren Durchführbarkeft und besseren Automatisierbarkeit Bei diesen Testverfahren tritt jedoch ein grundsätzliches Problem auf, wenn die Probenkonzentration des Analyten sehr hoch ist. Sehr hohe Probenkonzentrationen sind z.B. von Analyten wie dem Albumin, lmmunglobulinen, β2-Mikrogiobulin, Huma-Choriongonadotropin (hCG), Ferritin, Aipha-Fetoprotein (AFP) bekannt. Wird nichtgebundener Analyt nicht wie im Zweischritt-Sandwichimmunoassay vor der Inkubation mit dem gelabelten Antikörper entfernt, kann es dazu kommen, daß die Bindungsstellen der Antikörper abgesättigt sind, ohne daß es zur Ausbildung eines Sandwich komplexes gekommen ist. Dies hat zur Folge, daß das Meßsignal bei Proben mit steigender Analytkonzentration zunächst zunimmt und dann ab einer gewissen Grenzkonzentration wieder abnimmt (s. z.B. Fig. 1 in Papik et al. (1999) Clin. Chem. Lab. Med., 37:471-476). Dieses Phänomen, welches auch als "prozone effect" oder "High-dose-hook-Effekt" bekannt ist und nachfolgend "Hook-Effekt" genannt wird, führt dazu, daß das Meßsignal von Proben mit sehr hoher Analytkonzentration innerhalb des Standardkurvenbereichs gefunden wird und diesen Proben fälschlicherweise eine viel zu niedrige Analytkonzentration zugeordnet wird.

Es ist daher wichtig, den Einschritt-Immunoassay bzw. den homogenen Sandwichlmmunoassay so zu gestalten, daß die Grenzkonzentration möglichst weit in den hohen Analytkonzentrationsbereich oder besser über den natürlich vorkommenden Analytkonzentrationsbereich hinaus verschoben wird. Eine weitere bzw. alternative Maßnahme ist, einen Verfahrensschritt einzuführen, der eine Hook-Probe anzeigt, so daß nach entsprechender Probenverdünnung die Analytkonzentration ermittelt werden kann.

Papik et al. schlagen bei einem turbidimetrischen Test zur Lösung dieser Aufgabe die Analyse der Reaktionskinetik mittels eines sehr aufwendigen Rechenverfahrens vor.

In EP-A1-0 787 986 wird vorgeschlagen in solchen Testen affinitätschromatographisch gereinigte polyklonale Antikörper einzusetzen. Dieses Verfahren hat jedoch den Nachteil, daß es nicht bei allen Testverfahren, insbesondere nicht bei nicht-immunchemischen Testverfahren, anwendbar ist.

Ferner stehen nicht für alle nachzuweisenden Analyten polyklonale Antikörper zur Verfügung.

US 4,590,169 und US 4,595,661 beschreiben den Einsatz von Antikörpern mit unterschiedlicher Affinität zur Verringerung des Hook-Efektes. Gemäß US 4,595,661 wird die Probe mit einem an eine Polystyrolkugel gebundenen Antikörper und zwei Meerrettichperoxidase-Antikörper-Konjugaten inkubiert, wobei die drei Antikörper an unterschiedliche Epitope auf dem nachzuweisenden Protein binden und die beiden Enzym-markierten Antikörper eine voneinander deutlich unterschiedliche Affinität aufweisen. Nach der Inkubation werden die ungebundenen Substanzen durch Waschen entfernt und die an die Polystyrolkugel gebundene Enzymaktivität gemessen. Ein Nachteil dieser Verfahren besteht darin, daß Antikörper unterschiedlicher Affinität benötigt werden. Ein weiterer Nachteil ist die verringerte Nachweisempfindlichkeit solcher Teste, da der Einsatz von Konjugaten mit niederaffinen Antikörpern aufgrund höherer unspezifischer Bindung an die Festphase zu einer Erhöhung des Hintergrundsignals führt.

In EP-A2-0 617 285 ist ein homogener turbidimetrischer hCG-Test beschrieben, bei dem zur Verringerung des Hook-Effektes die Probe zunächst mit einem löslichen Anti-hCG-Antikörperfragment, welches mindestens zwei Bindungsstellen für den Analyten besitzt, inkubiert wird, dann an Latexpartikel gebundene Antikörperfragmente von Anti-hCG-Antikörpem mit einer anderen Epitopspezifität zugesetzt werden und die Absorptionsänderung gemessen wird. Die Zugabe des ungelabelten löslichen Antikörpers bewirkt eine zusätzliche Quervemetzung der Latexpartikel und dadurch eine Verschiebung des Hook-Effektes in Richtung höherer Analytkonzentrationen. Homogene turbidimetrische Meßverfahren haben jedoch den Nachteil, daß sie die für bestimmte Parameter benötigte Nachweisempfindlichkeit nicht aufweisen.

Für den Fachmann stellt sich daher die Aufgabe, Nachweisverfahren, insbesondere solche die eine hohe Nachweisempfindlichkeit aufweisen, so zu entwickeln, daß ein Hook-Effekt vermieden, verringert und/oder zumindest erkannt wird.

Diese Aufgabe wird durch die Bereitstellung des erfindungsgemäßen Verfahren gemäß Anspruch 1 gelöst. Bei diesem Verfahren zum quantitativen oder qualitativen Nachweis eines Analyten A in einer Probe wird die Probe zum Nachweis, zur Vermeidung und/oder zur Verminderung des Hook-Effektes mit einem an eine Festphase assoziierten Analyt A-spezifischen Bindungspartner R1, einem Analyt A-spezifischen Bindungspartner R2, der mit einem Label L1 assoziiert ist, und einem Analyt A-spezifischen Bindungspartner R3, der mit einem Label L2 assoziiert ist, inkubiert. Die Bindungspartner R2 und R3 werden so ausgewählt, daß die Absättigung der Analyt-A-Bindungsstellen der im Inkubationsansatz befindlichen Bindungspartner R2 im Vergleich zur Absättigung der Analyt-A-Bindungsstellen der im Inkubationsansatz befindlichen Bindungspartner R3 bei einer höheren Analyt A-Konzentration und/oder zu einem späteren Inkubationszeitpunkt erfolgt. Das von L1 abhängige Meßsignal wird von dem von L2 oder von L1 plus L2 abhängigen Meßsignal entweder zeitlich voneinander getrennt oder mit Hilfe eines anderen Meßverfahrens bestimmt. Bevorzugt wird jeweils das Meßsignal von mit den gebildeten Sandwich-Komplexen assoziierten Labein gemessen.

Das erfindungsgemäße Testverfahren kann unabhängig von der Reihenfolge der Zugabe von R1, R2 und R3 oder der jeweiligen Inkubationsdauer von Probe und dem jeweiligen Bindungspartner ausgeführt werden. Auch können ungebundene Reagenzkomponenten und/oder Probenbestandteile durch einen Trenn- oder Waschschritt entfernt werden bevor das oder die Meßsignale gemessen werden. Ein solcher Trenn- und/oder Waschschritt kann z.B. auch nach der Messung des von L1-abhängigen Meßsignals erfolgen oder vor der Messung aller Meßsignale.

Die Meßsignale können z.B. jeweils als Einzelmeßwert, Mittelwert, Median oder Summe von mehreren Einzelmeßwerten und/oder in Form von Kinetiken erfaßt werden. Mit unterschiedlichen Meßverfahren sind Verfahren gemeint, die das von L1 abhängige Meßsignal unabhängig von dem von L2 oder von L1 plus L2 abhängigen Meßsignal erfassen können. Zum Beispiel könnte es sich beim Label L1 um einen Mikropartikel handeln, der mit einem nephelometrischen Verfahren gemessen werden kann, und beim Label L2 um ein Enzym, dessen Aktivität z.B. photometrisch bestimmt wird. Bei einem anderen Testsystem kann es sich bei L1 und L2 um zwei Fluoreszenzlabel handeln, deren Fluoreszenz jeweils bei einer anderen Wellenlänge gemessen wird. Zur weiteren Auswertung kann beispielsweise das Verhältnis zwischen dem "L1-Wert" und dem "L2-Wert" bzw. dem "L2 plus L1-Wert" gebildet werden, wobei unter dem Begriff "Wert" direkt das Meßsignal oder ein auf Basis des Meßsignals weiter überarbeiteter, dem Meßsignal proportionaler Wert gemeint sein kann. Das so ermittelte Verhältnis kann mit für den Test spezifischen Kenngrößen verglichen werden, so daß die den Test durchführende Person oder der Automat schnell erkennen kann, ob ein Hook-Effekt vorliegt und/oder ob die Probe in einer geeigneten Verdünnung erneut gemessen werden sollte.

Das Verfahren beruht darauf, daß die Ausbildung der Sandwich-Komplexe R1-A-R2 und R1-A-R3 durch den Hook-Effekt unterschiedlich stark beeinflußt wird: Ein abnehmendes Meßsignal tritt bei der Sandwich-Komplexbildung R1-A-R2 im Vergleich zur Sandwich-Komplexbildung R1-A-R3 erst bei einer höheren Analytkonzentration der Probe auf. Die geeigneten Bindungspartner und Reaktionsbedingungen können vom Fachmann relativ einfach ermittelt werden, indem er die Sandwich-Komplexbildung R1-A-R2 und die Sandwich-Komplexbildung R1-A-R3 zunächst jeweils in getrennten Testverfahren mißt.

Bei einer bevorzugten Ausführungsform der Erfindung handelt es sich beim Bindungspartner R2 und R3 um den gleichen Bindungspartner. Hierbei wird R2 in Form von R2-Aggregaten und/oder vielen R2-Molekülen, die mit einer suspendierbaren Festphase assoziiert sind, eingesetzt, während R3 zumindest in der Anfangsphase der Inkubation als sofitäres Molekül eingesetzt wird.

R2 und R3 können auch verschiedene Bindungspartner sein. Zum Beispiel kann ein Bindungspartner als R2 eingesetzt werden, der gegen eine einzeln vorhandene Bindungsstelle auf dem Analyten gerichtet ist, während R3 ein mehrfach vorhandene Bindungsstelle auf dem Analyten erkennt. Ferner kann ein Bindungspartner R2 eingesetzt werden, dessen Bindefähigkeit an den Analyten schwächer ausgeprägt ist als die des Bindungspartners R3.

In der Regel wird die Nachweisempfindlichkeit, d.h. die noch sicher erfaßbare kleinste Analytkonzentration, des Testsystems Festphase-R1 und Label-R2 geringer sein als die Nachweisempfindlichkeit des Testsystems Festphase-R1 und Label-R3.

Allerdings wird das Testsystem Festphase-R1 und Label-R2 noch bei einer höheren Analytkonzentration richtige Konzentrationswerte anzeigen. Mit anderen Worten ausgedrückt deckt das Testsystem "Festphase-R1 und Label-R2" den oberen Meßbereich besser ab und das Testsystem "Festphase-R1 und Label-R3" deckt besser den unteren Meßbereich ab. Durch die getrennte Erfassung des zur R1-A-R2-Komplexbildung proportionalen L1-Meßsignals von dem von L2 oder von L1 plus L2 abhängigen Meßsignal ist es möglich, den Meßbereich des erfindungsgemäßen Testverfahrens zu erweitern und/oder einen Hook-Effekt zu erkennen. Liegt beispielsweise das L1-Meßsignal oberhalb des höchsten Standardkurvenwertes und das L2-Meßsignal innerhalb der Standardkurve, auch Eichkurve genannt, dann ist das ein sicheres Indiz für eine "High-dose-hook-Probe". Im Falle eines erkannten Hook-Effektes wird die Probe in entsprechender Verdünnung neu getestet und die richtige Analytkonzentration ermittelt.

Im Gegensatz zu den bisher bekannten Verfahren ist das erfindungsgemäße Verfahren auch über das Gebiet der Immunoassays hinaus breit anwendbar, z.B. bei Bindungstesten mit Nukleinsäuremolekülen als spezifische Bindungspartner. Es werden beim erfindungsgemäßen Verfahren weder besonders aufgereinigte Antikörper noch Antikörper mit unterschiedlicher Affinität benötigt. Im Gegenteil, in einer bevorzugten Variante des erfindungsgemäßen Verfahrens handelt es sich bei R2 und R3 um den gleichen spezifischen Bindungspartner. Ferner läßt sich das erfindungsgemäße Verfahren leichter automatisieren.

Ein weiterer Vorteil des erfindungsgemäßen Verfahrens ist die Möglichkeit der Meßbereichserweiterung: So kann beispielsweise das L1-abhängige Meßsignal genutzt werden, um die Konzentration von Proben mit höherer Konzentration zu ermitteln, während das von L2-abhängige Meßsignal genutzt wird, um die Konzentration von Proben mit niedriger Konzentration zu ermitteln. Es müssen folglich weniger Proben verdünnt werden.

Durch die entsprechende Auswahl von L1 und L2 und/oder der anderen Verfahrensparameter, z.B. die R2- und/oder die R3-Konzentration im Inkubationsansatz, ist es möglich, das erfindungsgemäße Verfahren so zu optimieren, daß trotz des großen Meßbereichs und der sicheren Hook-Effekt-Erkennung die unspezifische Festphasenbindung von R2-L1 die über das System R1-Festphase und R3-L2 gewährleistete Testsensitivität nicht beeinflußt.

Besonders vorteilhaft läßt sich das erfindungsgemäße Verfahren auch bei Analyten einsetzen, die aufgrund von mit den spezifischen Bindungspartnem kreuzreagierenden Analyten nur sehr schwer mit einem homogenen und/oder einem Einschritt-Sandwichassay zu bestimmen sind. So ist es üblich LH (Luteinisierendes Hormon) mit einem Immunoassay zu bestimmen bei dem Antikörper gegen die α-Kette und Antikörper gegen den LH-spezifischen Teil der β-Kette eingesetzt werden. Ist in der Probe zufälligerweise hCG in großer Menge vorhanden, kann dies bei einem Einschritt-Sandwichtest und/oder einem homogenen Test dazu führen, daß alle oder fast alle Bindungstellen der gegen die α-Kette gerichteten Antikörper durch hCG-Moleküle blockiert sind. Der Antikörper-LH-Antikörper-Sandwichkomplex kann sich folglich nicht ausbilden und die LH-Probenkonzentrationen wird falsch ermittelt. Mit Hilfe des erfindungsgemäßen Verfahren ist es nun möglich solche Analyten nicht wie üblich mit einem Zweischritt-Sandwichtest zu messen sondern mit dem viel schnelleren Einschritt-Sandwichtest: Hiebei wird R1 so ausgewählt, daß dieser Bindungspartner den Analyten und die kreuzreagierenden Substanzen gleich gut erkennt, und R2 und R3 erkennen jeweils spezifisch entweder den Analyten oder die kreuzreagierenden Substanzen. Um das weiter oben beschriebene Beispiel zur exemplarischen Beleuchtung der Erfindung nochmals aufzugreifen, würde man erfindungsgemäß z.B. gegen die α-Kette gerichtete Antikörper als R1, spezifische Anti-hCG-Antikörper als R2 und spezifische Anti-LH-Antikörper als R3 einsetzen. Das von der R1-hCG-R2-Sandwichbüdung abhängige L1-Signal zeigt an, ob die Probe zur korrekten LH-Konzentrationsbestimmung z.B. in einem Zweischrittlmunoassay nochmals gemessen werden muß oder nicht. Dieses erfindungsgemäße Verfahren läßt sich auch entsprechend zur Messung von Antikörpern, insbesondere zur Messung von Antikörpern einer bestimmten Immunglobulinklasse wie z.B. lgM, einsetzen, die gegen ein bestimmtes, beispielsweise virales oder bakterielles Antigen gerichtet sind, wobei die anderen in der Probe vorhandenen Antikörper im großen Überschuß vorhanden sein können. Diese oben beschriebene Verwendung des erfindungsgemäßen Verfahrens soll im Sinne der Erfindung im Ausdruck "zum Nachweis, zur Vermeidung und/oder zur Verminderung des Hook-Effektes" miteingeschlossen sein.

Bevorzugte Ausführungsformen der Erfindung sind in den Ansprüchen 2-19 beschrieben. Bevor diese und andere bevorzugte Ausführungsformen der Erfindung weiter erläutert werden, sollen zum leichteren Verständnis der Erfindung einige Begriffe näher erläutert werden:

Bei einem "quantitativen Nachweis" wird die Menge oder die Konzentration des Analyten in der Probe gemessen. Von dem Begriff des "quantitativen Nachweises" sind auch semiquantitative Methoden umfaßt, die nur die ungefähre Menge oder Konzentration des Analyten in der Probe erfassen oder nur zu einer relativen Mengen- oder Konzentrationsangabe dienen können. Unter einem "qualitativen Nachweis" ist der Nachweis des Vorhandenseins oder Fehlens des Analyten in der Probe überhaupt oder das Anzeigen, daß die Konzentration des Analyten in der Probe unterhalb oder oberhalb eines bestimmten oder mehrerer bestimmter Schwellenwerte liegt, zu verstehen.

Unter dem Begriff "Analyt" ist die im Testverfahren nachzuweisende Substanz zu verstehen. Beispiele für einen Analyten sind in EP-A2-0 515 194 auf den Seiten 8-15 aufgeführt. Der Analyt kann ein Mitglied eines spezifischen Bindungspaars sein. Der Analyt mag eine Bindungsstelle (monovalent, üblicherweise ein Hapten) oder mehrere Bindungsstellen (polyvalent) aufweisen. Bei immunchemischen Testen wird eine solche Bindungsstelle häufig auch als Epitop bezeichnet. Ferner kann der Analyt eine einzelne Substanz sein oder eine Gruppe von Substanzen, die wenigstens eine einzige gemeinsame Bindungsstelle aufweisen.

Ein monovalenter Analyt hat üblicherweise ein Molekulargewicht von etwa 100 bis 2000, insbesondere von 125 bis 1000. Viele Oligopeptide, Oligonukleotide, Oligosacharide, Arzneimittel, Drogen, Metaobolite, Pestizide etc. werden vom Begriff eines monovalenten Analyten umfaßt. Ein poylvalenter Analyt hat üblicherweise ein Molekulargewicht von über 5000, meistens Ober 10 000. Beispiele für polyvalente Analyte sind Polypeptide, Polysacharide, Nukleinsäuren, Zellen, Zeilbestandteile inklusive Chromosomen, Gene, Mitochondrien und andere Zellorganelle, Zellmembranen, etc. Häufig sind Proteine die nachzuweisenden Substanzen. Solche Proteine mögen Mitglieder einer Proteinfamilie sein, deren Mitglieder durch ähnliche strukturelle Merkmale und/oder durch eine ähnliche biologische Funktion gekennzeichnet sind. Beispiele für analytisch interessante Proteinfamilien sind Proteine von Krankheitserregern, Immunogiobuline, Cytokine, Enzyme, Hormone, Tumormarker, Stoffwechselmarker, gewebespezifische Antigene, Histone, Albumine, Globuline, Skleroproteine, Phosphoproteine, Mucine, Chromoproteine, Lipoproteine, Nukleoproteine, Glykoproteine, Proteoglykane, Rezeptoren, HLA, Gerinnungsfaktoren, Herzinfarktmarker, etc. Andere analytisch interessante Substanzen sind beispielsweise einzel- oder doppelsträgige Oligo- und Polynukleotide

Unter einer "Probe" ist im Sinne der Erfindung das Material zu verstehen, das die nachzuweisende Substanz ("Analyt") vermutlich enthält. Der Begriff Probe umfaßt beispielsweise biologische Flüssigkeiten oder Gewebe insbesondere von Menschen und Tieren wie Blut, Plasma, Serum, Sputum, Exudat, bronchoalveoläre Lavage, Lymphflüsigkeit, Synovialflüssigkeit, Samenflüssigkeit, Vaginalschleim, Feces, Urin, Liquor, Haare, Haut, Gewebeproben oder -schnitte. Ferner werden umfaßt Zellkulturproben, pflanzliche Flüssigkeiten oder Gewebe, forensische Proben, Wasser- und Abwasserproben, Nahrungsmittel, Arzneimittel. Gegebenenfalls müssen die Proben vorbehandelt werden, um den Analyten für das Nachweisverfahren zugänglich zu machen oder um störende Probenbestandteile zu entfemen. Solche Vorbehandlung von Proben mag die Abtrennung und/oder Lyse von Zellen beinhalten, das Präzipitieren, die Hydrolyse oder die Denaturierung von Probenbestandteilen wie z.B. Proteinen, die Zentrifugation von Proben, das Behandeln der Probe mit organischen Lösungsmitteln wie z.B. Alkohole, insbesondere Methanol; das Behandeln der Probe mit Detergenzien. Häufig wird die Probe in ein anderes, meistens wässriges, Medium überführt, welches mit dem Nachweisverfahren möglichst nicht interferieren soll. Der Analyt kann auch amplifiziert werden. Eine Amplfizierung von Nukleinsäuren führt beispielsweise zur Generierung von einer oder mehreren Kopien der nachzuweisenden Nukleinsäurekette. Solche Amplifizierungsmethoden sind dem Fachmann gut bekannt, z.B. "polymerase chain reaction" (PCR), "ligase chain reaction" (LCR), "amplification using Q beta replicase", "nucleic acid sequence based amplification" (NASBA), "single primer amplification" (ASPP) und andere.

Unter einem "spezifischen Bindungspartner" ist ein Mitglied eines spezifischen Bindungspaars zu verstehen. Bei den Mitgliedern eines spezifischen Bindungspaars handelt es sich um zwei Moleküle, die jeweils mindestens eine zu einer Struktur des anderen Moleküls komplementäre Struktur aufweisen, wobei die beiden Moleküle sich über eine Bindung der komplementären Strukturen zu binden vermögen. Der Begriff Molekül umfaßt auch Molekülkomplexe wie z.B. Enzyme, die aus Apo- und Coenzym bestehen, Proteine, die aus mehreren Untereinheiten bestehen, Lipoproteine bestehend aus Protein und Lipiden, etc.. Spezifische Bindungspartner können natürlich vorkommende aber auch z.B. mittels chemischer Synthese, mikrobiologischer Techniken und/oder gentechnologischer Verfahren hergestellte Substanzen sein. So lassen sich mittlerweile mit Hilfe von Phage Display Bibliotheken, über synthetische Peptiddatenbanken oder mittels "Recombinatorial Antibody Libraries" spezifische Bindungspartner selektieren (Larrick & Fry (1991) Human Antibodies and Hybridomas, 2:172-189). Zur Illustration des Begriffs spezifischer Bindungspartner, aber nicht als Einschränkung zu verstehend, sind z.B. zu nennen: Thyroxinbindendes Globulin, steroidbindende Proteine, Antikörper, Antigene, Haptene, Enzyme, Lektine, Nukleinsäuren, Repressoren, Oligo- und Polynukleotide, Protein A, Protein G, Avidin, Streptavidin, Biotin, Komplementkomponente C1q, nukleinsäurebindende Proteine, etc.. Spezifische Bindungspaare sind beispielsweise: Antikörper-Antigen, Antikörper-Hapten, Operator-Repressor, Nuclease-Nukleotid, Biotin-Avidin, Lektin-Polysaccharid, Steroid-steroidbindendes Protein, Wirkstoff-Wirkstoffrezeptor, Hormon-Hormonrezeptor, Enzym-Substrat, lgG-Protein A, komplementäre Oligo- oder Polynukleotide, etc..

Unter dem Begriff "Antikörper" ist im Sinne dieser Erfindung ein Immunglobulin, z.B. ein Immunglobulin der Klasse bzw. Subklasse IgA, IgD, IgE, IgG₁, IgG₂ₐ, IgG_{2b}, IgG₃, IgG₄, IgM, zu verstehen. Ein Antikörper weist mindestens eine Bindungsstelle (häufig Paratop genannt) für ein Epitop (häufig auch antigene Determinante genannt) auf einem Antigen oder Hapten auf. Ein solches Epitop ist z.B. durch seine räumliche Struktur und/oder durch das Vorhandensein von polaren und/oder apolaren Gruppen gekennzeichnet. Die Bindungsstelle des Antikörpers ist komplementär zum Epitop. Die Antigen-Antikörper-Reaktion bzw. die Hapten-Antikörper-Reaktion funktioniert nach dem sogenannten "Schlüssel-Schlüsselloch-Prinzip", und ist in der Regel in einem hohen Grad spezifisch, d.h. die Antikörper vermögen kleine Abweichungen in der Primärstruktur, in der Ladung, in der räumlichen Konfiguration und der sterischen Anordnung des Antigens oder Haptens zu unterscheiden. Insbesondere die sogenannten "complementarity determining regions" des Antikörpers tragen zur Bindung des Antikörpers an das Antigen oder Hapten bei.

Der Begriff "Antigene" umfaßt monovalente und polyvalente Antigene. Ein polyvalentes Antigen ist ein Molekül oder ein Molekülkomplex, an das/den mehr als ein Immunglobulin gleichzeitig binden kann, während bei einem monovalenten Antigen jeweils nur ein einziger Antikörper zur selben Zeit binden kann. Als Hapten wird üblicherweise ein Molekül bezeichnet, das nicht für sich allein immunogen ist, sondern das zu lmmunisierungszwecken üblicherweise an einen Träger gebunden wird.

Unter dem Begriff Antikörper sind im Sinne dieser Erfindung nicht nur komplette Antikörper zu verstehen sondern ausdrücklich auch Antikörperfragmente, wie z.B. Fab, Fv, F(ab')₂, Fab'; sowie auch chimäre, humanisierte, bi- oder oligospezifische, oder "single chain" Antikörper; des weiteren auch Aggregate, Polymere und Konjugate von Immunglobulinen und/oder deren Fragmenten, sofern die Bindungseigenschaften an das Antigen oder Hapten erhalten sind. Antikörperfragmente lassen sich beispielsweise durch enzymatische Spaltung von Antikörpern mit Enzymen wie Pepsin oder Papain herstellen. Antikörperaggregate, - polymere und -konjugate können durch vielfältige Methoden generiert werden, z.B. durch Hitzebehandlung, Umsetzung mit Substanzen wie Glutaraldehyd, Reaktion mit immunglobulin-bindenden Molekülen, Biotinylierung von Antikörpern und anschließende Reaktion mit Streptavidin oder Avidin, etc..

Bei einem Antikörper im Sinne dieser Erfindung kann es sich um einen monoklonalen oder um einen polyklonalen Antikörper handeln. Der Antikörper kann nach den üblichen Verfahren hergestellt worden sein, z.B. durch lmmunisierung des Menschen oder eines Tieres, wie z.B. Maus, Ratte, Meerschweichen, Kaninchen, Kamel, Pferd, Schaf, Ziege, Huhn (s.a. Messerschmid (1996) BIOforum, 11:500-502), und anschließender Gewinnung des Antiserums; oder durch die Etablierung von Hybridomazellen und der anschließenden Reinigung der sekretierten Antikörper, oder durch Klonierung und Expression der Nukleotidsequenzen bzw. modifizierter Versionen davon, die die Aminosäuresequenzen kodieren, die für die Bindung des natürlichen Antikörpers an das Antigen und/oder Hapten verantwortlich sind. Antikörper sind auch herstellbar ggf. unter Zuhilfenahme gentechnologischer Methoden in pflanzlichen - wie z.B. Hefezellen - (Fischer et al. (1999) Biol. Chem., 380:825-839; Hiatt et al. (1992) Genetic Engineering, 14:49-64)), tierischen und prokaryontischen Zellen (s. z.B. WO 95/25172) sowie isolierten menschlichen Zellen.

Der Begriff "Festphase" im Sinne dieser Erfindung beinhaltet einen Gegenstand, der aus porösem und/oder nicht porösem, in der Regel wasserunlöslichem Material besteht und die unterschiedlichsten Formen aufweisen kann, wie z,B. Gefäß, Röhrchen, Mikrotitrationsplatte, Kugel, Mikropartikel, Stäbchen, Streifen, Filter- oder Chromatographiepapier, etc. In der Regel ist die Oberfläche der Festphase hydrophil oder kann hydrophil gemacht werden. Die Festphase kann aus den unterschiedlichsten Materialien bestehen wie z.B. aus anorganischen und/oder aus organischen Materialien, aus synthetischen, aus natürlich vorkommenden und/oder aus modifizierten natürlich vorkommenden Materialien. Beispiele für Festphasenmaterialien sind Polymere wie z.B. Zellulose, Nitrozellulose, Zelluloseacetat, Polyvinylchlorid, Polyacrylamid, vemetzte Dextranmoleküle, Agarose, Polystyrol, Polyethylen, Polypropylen, Polymethacrylat oder Nylon; Keramik; Glass; Metalle, insbesondere Edelmetalle wie Gold und Silber; Magnetit; Mischungen oder Kombinationen derselben; etc. Auch Zellen, Liposomen oder Phospholipidvesikel, sind vom Begriff Festphase miterfaßt.

Die Festphase kann einen Überzug aus einer oder mehreren Schichten aufweisen, z.B. aus Proteinen, Kohlehydraten, lipophilen Substanzen, Biopolymeren, organischen Polymeren oder Mischungen hiervon, um beispielsweise die unspezifische Bindung von Probenbestandteilen an die Festphase zu unterdrücken oder zu verhindem, oder um beispielsweise Verbesserungen zu erreichen hinsichtlich der Suspensionsstabilität von partikulären Festphasen, der Lagerstabilität, der formgebenden Stabilität oder der Resistenz gegen UV-Licht, Mikroben oder sonstige zerstörend wirkender Agenzien.

Der Begriff "assoziiert" ist breit zu verstehen und umfaßt beispielsweise eine kovalente und eine nicht-kovalente Bindung, eine direkte und eine indirekte Bindung, die Adsorption an eine Oberfläche und den Einschluß in eine Vertiefung oder einen Hohlraum, etc.. Bei einer kovalenten Bindung sind die Antikörper oder Bindungspartner über eine chemische Bindung an die Festphase oder an das Label gebunden. Üblicherweise spricht man von einer kovalenten Bindung zwischen zwei Molekülen, wenn wenigstens ein Atomkem des einen Moleküls Elektronen teilt mit wenigstens einem Atomkem des zweiten Moleküls. Beispiele für eine nicht-kovalente Bindung sind die Oberflächenadsorption, der Einschluß in Hohlräume oder die Bindung von zwei spezifischen Bindungspartnem. Neben einer direkten Bindung an die Festphase oder das Label können die Antikörper oder Bindungspartner auch indirekt über spezifische Wechselwirkung mit anderen spezifischen Bindungspartnern an die Festphase oder das Label gebunden sein (s.a. EP-A2-0 411 945). Dies soll anhand von Beispielen näher illustriert werden: Der biotinylierte Antikörper kann über labelgebundenes Avidin an das Label gebunden werden; oder es kann ein Fluorescein-Antikörperkonjugat über festphasengebundene Anti-Fluorescein-Antikörper an die Festphase gebunden werden; oder der Antikörper kann über Immunglobulin-bindende Proteine an die Festphase oder das Label gebunden werden.

Bei einem "signalbildenden System" kann es sich um eine oder mehrere Komponenten handeln, wobei es sich wenigstens bei einer Komponente um ein nachweisbares label handelt. Als Label ist jedes Molekül zu verstehen, das selbst ein Signal produziert oder die Produktion eines Signals induzieren kann wie z.B. eine fluoreszierende Substanz, eine radioaktive Substanz, ein Enzym, oder eine chemilumineszierende Substanz. Das Signal kann beispielsweise anhand der Enzymaktivität, der Lumineszenz, der Lichtabsorption, der Lichtstreuung, der ausgestrahlten elektromagnetischen oder radioaktiven Strahlung, oder einer chemischen Reaktion nachgewiesen oder gemessen werden.

Ein "Label" vermag selbst ein nachweisbares Signal zu erzeugen, so daß keine weiteren Komponenten notwendig sind. Viele organische Moleküle absorbieren ultraviolettes und sichtbares Licht, wobei durch die Lichtabsorption übertragene Energie diese Moleküle in einen angeregten Energiezustand kommen können, und die absorbierte Energie in Form von Licht einer anderen Wellenlänge als der des eingestrahlten Lichts abgeben. Wieder andere Label können direkt ein nachweisbares Signal erzeugen wie z.B. radioaktive lsotope, Farbstoffe oder magnetische und nicht-magnetische Mikropartikel.

Wieder andere Label benötigen zur Signalerzeugung weitere Komponenten, d.h. das signalproduzierende System schließt in einem solchen Fall all die für die Signalbildung benötigten Komponenten mit ein wie z.B. Substrate, Coenzyme, Quencher, Beschleuniger, zusätzliche Enzyme, Substanzen die mit Enzymprodukten reagieren, Katalysatoren, Aktivatoren, Cofaktoren, Inhibitoren, Ionen etc..

Geeignete Label (s.a. EP-A2-0 515 194; US 5,340,716; US 5,545,834; Bailey et al. (1987) J. Pharmaceutical & Biomedical Analysis 5:649-658) sind beispielsweise Enzyme einschließlich Meerrettichperoxidase, alkalische Phosphatase, Glukose-6-Phosphatdehydrogenase, Alkoholdehydrogenase, Glucoseoxidase, β-Galactosidase, Luciferase, Urease und Acetylcholinesierase; Farbstoffe; fluoreszierende Substanzen einschließlich Fluoresceinisothiocyanat, Rhodamin, Phycoerythrin, Phycocyanin, Ethidiumbromid, 5-Dimethylaminonapthalen-1-sulfonylchlorid und fluoreszierende Chelate von seltenen Erden; chemilumineszierende Substanzen einschließlich Luminol, Isoluminol, Acridiniumverbindungen, Olefin, Enolether, Enamin, Arylvinylether, Dioxen, Arylimidazol, Lucigenin, Luciferin und Aequorin; Sensitizer einschließlich Eosin, 9,10-Dibromoanthracen, Methylen Blau, Porphyrin, Phthalocyanin, Chlorophyll, Rose Bengal; Coenzyme; Enzymsubstrate; radioaktive lsotope einschließlich ¹²⁵I, ¹³¹I, ¹⁴C, ³H, ³²P, ³⁵S, ¹⁴C, ⁵¹Cr, ⁵⁹Fe, ⁵⁷Co und ⁷⁵Se; Partikel einschließlich magnetische Partikel oder Partikel, bevorzugt Latexpartikel, die selbst beispielsweise mit Farbstoffen, Sensitizem, fluoreszierenden Substanzen, chemilumineszierenden Substanzen, Isotopen oder anderen nachweisbaren Labeln markiert sein können; Solpartikel einschließlich Gold- oder Silbersolen; Liposomen oder Zellen, die selbst mit nachweisbaren Labeln markiert sein können; etc.

Ein signalbildendes System kann auch Komponenten umfassen, die bei räumlicher Nähe miteinander in eine nachweisbare Wechselwirkung treten können, z.B. in Form von Energiespendem und Energieempfängem wie beispielsweise Photosensitizer und chemolumineszierende Substanzen (EP-A2-0 515 194), Photosensitizer und Fluorophore (WO 95/06877), radioaktives lod¹²⁵ und Fluorophore (Udenfriend et al. (1985) Proc. Natl. Acad. Sci. 82:8672-8676), Fluorophore und Fluorophore (Mathis (1993) Clin. Chem. 39:1953-1959) oder Fluorophore und Fluoreszenz-Quencher (US 3,996,345).

Unter einer Wechselwirkung zwischen den Komponenten ist die direkte Übertragung von Energie zwischen den Komponenten, z.B. durch Licht- oder Elektronenstrahlung sowie über kurzlebige reaktive chemische Moleküle, miteingeschlossen. Ferner umfaßt sind hiervon auch Vorgänge, bei denen die Aktivität einer Komponente durch eine oder mehrere andere inhibiert oder verstärkt wird, beispielsweise die Hemmung oder Steigerung der Enzymaktivität oder die Hemmung, Steigerung oder Veränderung (z.B. Wellenlängenverschiebung, Polarisation) des von der beeinflußten Komponente ausgesendeten elektromagnetischen Strahlung. Die Wechselwirkung zwischen den Komponenten umfaßt auch Enzymkaskaden. ln diesem Fall sind die Komponenten Enzyme, von denen mindestens eines das Substrat für ein anderes liefert, so daß eine maximale oder minimale Reakionsgeschwindigkeit der gekoppelten Substratumsetzung resultiert.

Eine effektive Wechselwirkung zwischen den Komponenten findet in der Regel statt, wenn diese räumlich benachbart vorliegen, also z.B. innerhalb eines Abstandbereiches von wenigen µm, insbesondere innerhalb eines Abstandbereiches von unter 600 nm, bevorzugt unter 400 nm, ganz besonders bevorzugt von unter 200 nm.

Mikropartikel werden häufig als Festphase und/oder als Label genutzt. Unter dem Begriff "Mikropartikel" sind im Sinne dieser Erfindung Teilchen zu verstehen, die einen ungefähren Durchmesser von wenigstens 20 nm und nicht mehr als 20 µm aufweisen, üblicherweise zwischen 40 nm und 10 µm, bevorzugt zwischen 0,1 und 10 µm, besonders bevorzugt zwischen 0,1 und 5 µm, ganz besonders bevorzugt zwischen 0,15 und 2 µm. Die Mikropartikel können regelmäßig oder unregelmäßig geformt sein. Sie können Kugeln, Spheroide, Kugeln mit mehr oder weniger großen Kavitäten oder Poren darstellen. Die Mikropartikel können aus organischem, aus anorganischem Material oder aus einer Mischung oder Kombination von beiden bestehen. Sie können aus einem porösen oder nicht porösen, einem schwellfähigen oder nicht schwellfähigen Material bestehen. Prinzipiell können die Mikropartikel jegliche Dichte haben, bevorzugt sind jedoch Partikel mit einer Dichte, die der Dichte des Wassers nahekommt wie etwa 0,7 bis etwa 1,5 g/ml. Die bevorzugten Mikropartikel sind in wässrigen Lösungen suspendierbar und möglichst lange suspensionsstabil. Sie mögen durchsichtig, teilweise durchsichtig oder undurchsichtig sein. Die Mikropartikel können aus mehreren Schichten bestehen wie beispielsweise die sogenannten "core-and-shell" Partikel mit einem Kem und einer oder mehreren umhüllenden Schichten. Der Begriff Mikropartikel umfaßt beispielsweise Farbstoffkristalle, Metallsolen, Silica-Partikel, Glaspartikel, Magnetpartikel, Polymerteilchen, Öltropfen, Lipidpartikel, Dextran, und Proteinaggregate. Bevorzugte Mikropartikel sind in wässrigen Lösungen suspendierbare und aus wasserunlöslichem Polymermaterial bestehende Partikel, insbesondere aus substituierten Polyethylenen. Ganz besonders bevorzugt sind Latexpartikel z.B. aus Polystyrol, Acrylsäurepolymeren, Methacrylsäurepolymeren, Acrylnitril-Polymeren, Acrylnitril-Butadien-Styrol, Polyvinylacetat-Acrylat, Polyvinylpyridin, Vinylchlorid-Acrylat. Von besonderem Interesse sind Latexpartikel mit reaktiven Gruppen an ihrer Oberfläche wie beispielsweise Carboxyl-, Amino- oder Aldehydgruppen, die eine kovalente Bindung z.B. von spezifischen Bindungspartnem an die Latexpartikel erlauben. Die. Herstellung von Latexpartikeln ist beispielsweise in EP 0 080 614, EP 0 227 054 und EP 0 246 446 beschrieben.

Bevorzugt handelt es sich bei dem erfindungsgemäßen Verfahren um einen heterogenen oder um einen homogenen Bindungstest nach dem Sandwich-Format.

Bei "homogenen Bindungstesten" erfolgt keine Trennung zwischen freien und komplexgebundenen Analyten. Beispiele für homogene Immunoassays (s.a. Boguslaski & Li (1982) Applied Biochemistry and Biotechnology, 7:401-414) sind viele turbidimetrische oder nephelometrische Methoden, wobei die zum Nachweis verwendeten spezifischen Bindungspartner mit Latexpartikel assoziiert sein können; EMIT®-Teste; CEDIA®-Teste; Fluoreszenz-Polarisations-Immunoassays; Luminescent Oxygen Channeling Immunoassays (EP-A2-0 515 194; Ullman et al. (1994) Proc. Natl. Acad. Sci., 91:5426-5430; Ullman et al. (1996) Clinical Chemistry ,42:1518 - 1526); etc.. Bei den sogenannten Gensondentesten sind die spezifischen Bindungspartner in der Regel Nukleinsäureketten, die zumindest teilweise komplementär sind zu Abschnitten der nachzuweisenden Nukleinsäurekette.

"Heterogene Bindungsteste" sind durch einen oder mehrere Trennungsschritte und/oder Waschschritte gekennzeichnet. Die Trennung kann beispielsweise durch Immunfällung, Fällung mit Substanzen wie Polyethylenglykol oder Ammoniumsulfat, Filtration, magnetische Abtrennung, Anbindung an eine Festphase wie z.B. an ein Röhrchen, an eine Kugel, an eine Mikrotitrationsplattenvertiefung, oder an ein Filter- oder Chromatographiepapier, erfolgen.

Bei "heterogenen Bindungstesten nach dem Sandwich-Format" wird der Analyt üblicherweise von einem Festphasen-assoziierten spezifischen Bindungspartner und einem spezifischen Bindungspartner, der mit einer Komponente eines signalbildenden System assoziiert ist, gebunden. Es kann sich hierbei um unterschiedliche oder um die gleichen spezifischen Bindungspartner handeln, z.B. kann bei einem Sandwich-Immunoassay ein Analyt-spezifischer monoklonaler Antikörper sowohl als Fänger (z.B. als Festphasenantikörper) als auch als markierter Antikörper eingesetzt werden, wenn der Analyt mehr als ein Epitop für diesen Antikörper aufweist. Bei den spezifischen Bindungspartnem kann es sich im Fall eines Sandwich-Immunassays um Analyt-spezifische Antikörper handeln oder, wenn der Analyt selbst ein Antikörper ist, um das Antigen und/oder um ein madi-fiziertes Antigen", z.B. einem gelabelten Antigen, einem Antigenteilstück, einem Antigenanalogon. Beispiele für solche Sandwichkomplexe sind: Festphase-Antikörper<>Analyt<>Antikörper-Label oder Festphase-Antigen<>Analyt(=Antikörper)<>Antigen-Label.

Eine besondere Ausführungsform eines heterogenen Immunoassays ist im Sinne dieser Erfindung der "indirekte Immunoassay": Der Analyt ist in diesem Fall ein Antikörper. Einer der spezifischen Bindungspartner ist dessen Antigen und/oder ein modifiziertes Antigen und der andere spezifische Bindungspartner ist in der Regel ein Antikörper, der den Analyten bindet, und/oder ein lmmunglobulin-bindendes Protein. Beispiele für solche Komplexe, die bei einem indirekten lmmunoassay gebildet werden können, sind: Festphase-Anti-IgM-Antikörper<>Analyt(=Anti-HbsAg-IgM)<>HBsAg-Label oder Festphase-HBsAg<>Analyt(=Anti-HbsAg-lgG)<>Protein A--Label.

Die oben beschriebenen Sandwich-Teste, inklusive der indirekten Immunoassays, können auch als homogene Testverfahren durchgeführt werden (s.a. EP-A2-0 515 194).

Eine bevorzugte Ausführungsform des erfindungsgemäßen Verfahrens ist, dadurch gekennzeichnet, daß (i) die Probe mit einem an eine Festphase assoziierten Analyt A-spezifischen Bindungspartner R1, einem Analyt A-spezifischen Bindungspartner R2, der mit einem Label L1 assoziiert ist, und einem Analyt A-spezifischen Bindungspartner R3, der mit einem Mitglied X eines spezifischen Bindungspaars assoziiert ist, inkubiert; (ii) zu einem späteren Zeitpunkt Label L2, welches mit dem zu X korrespondierenden Bindungspaarmitglied Y eines spezifischen Bindungspaars assoziiert ist, dem Testansatz hinzufügt; und (iii) das Meßsignal mindestens an zwei Zeitpunkten T1 und T2 bestimmt wird, wobei der frühere Zeitpunkt T1 spätestens kurz nach Zugabe von Label L2, welches mit Bindungspaarmitglied Y assoziiert ist, und der spätere Zeitpunkt T2 nach Zugabe von Label L2, welches mit Bindungspaarmitglied Y assoziiert ist, erfolgt.

Der mit dem Begriff "kurz nach Zugabe von Label L2" definierte Zeitraum ist so zu verstehen, daß der Zeitraum "Label L2-Zugabe bis Messung von L1" kurz ist im Vergleich zum Zeitraum "Label L2-Zugabe bis Messung von Label L2", d.h. bevorzugt weniger als 30%, besonders bevorzugt weniger als 15% des Zeitraums "Label L2-Zugabe bis Messung von Label L2".

In einem besonders bevorzugten Verfahren liegt der Zeitpunkt T1 vor der Zugabe von Label L2

Ein anderes bevorzugtes erfindungsgemäßes Verfahren ist dadurch gekennzeichnet, daß (i) die Probe mit einem an eine Festphase assoziierten Analyt A-spezifischen Bindungspartner R1, einem Analyt A-spezifischen Bindungspartner R2, der mit einem Label L1 assoziiert ist, und einem Analyt A-spezifischen Bindungspartner R3, der mit einem Mitglied X eines spezifischen Bindungspaars assoziiert ist, inkubiert; (ii) zu einem späteren Zeitpunkt Label L2, welches mit dem zu X korrespondierenden Bindungspaarmitglied Y eines spezifischen Bindungspaars assoziiert ist, dem Testansatz hinzufügt; und (iii) das Meßsignal von L1 und das Meßsignal von L2 mit Hilfe von unterschiedlichen Meßverfahren bestimmt.

Das Label L1 könnte z.B. ein Mikropartikel und das Label L2 ein Fluoreszenz oder Chemilumineszenzlabel sein, so daß das Meßsignal L1 turbidimetrisch oder nephelometrisch bestimmt werden könnte und das Meßsignal von L2 mit Hilfe eines Fluorometers oder Chemilumineszenzmeßgerätes. Bei einer anderen erfindungsgemäßen Ausführungsform könnten L1 und L2 z.B. unterschiedliche Fluoreszenzlabel sein, deren Fluoreszenzlicht jeweils bei einer anderen Wellenlänge detektiert wird.

Besonders bevorzugte Bindungspaare X<>Y für die oben beschriebenen Verfahren sind insbesondere Biotin<>Avidin, Biotin<>Streptavidin, oder Hapten<>Anti-Hapten-Antikörper wie z.B. Fluorescin<>Anti-Fluarescin, Digoxin<>Anti-Digoxin, oder Antigen<>Anti-Antigen-Antikörper wie z. B. Peroxidase<>Anti-Peroxidase, oder Nukleinsäure-Paare.

Eine besonders bevorzugte erfindungsgemäße Ausführungsform ist ein Test auf Basis des LOCl™-Verfahrens. Hierbei wird die Probe mit einem an einen Sensitizer Partikel assoziierten Analyt A-spezifischen Bindungspartner R1, einem Analyt A-spezifischen Bindungspartner R2, der mit einem Chemiluminescer-Partikel assoziiert ist, und einem Analyt A-spezifischen Bindungspartner R3, der mit einem Mitglied X eines spezifischen Bindungspaars, bevorzugt Biotin, assoziiert ist, inkubiert; (ii) zu einem späteren Zeitpunkt Chemilumniscer-Partikel, welche mit dem zu X korrespondierenden Bindungspaarmitgliedem Y, bevorzugt Streptavidin und/oder Avidin, eines spezifischen Bindungspaars assoziiert sind, dem Testansatz hinzufügt; und (iii) das Meßsignal mindestens an zwei Zeitpunkten T1 und T2 bestimmt wird, wobei der frühere Zeitpunkt T1 spätestens kurz nach Zugabe der Chemiluminescer Partikel, welche mit Bindungspaarmitgliedem Y assoziiert sind, und der spätere Zeitpunkt T2 nach Zugabe von Chemiluminescer-Partikeln, welche mit Bindungspaarmitgliedem Y assoziiert sind, erfolgt. Die mit dem Sensitizer-Partikel assoziierten Sensitizer-Moleküle können im angeregten Zustand Singlet-Sauerstoff erzeugen. Dieser Singlet-Sauerstoff kann mit den Chemilumineszenzverbindungen, die mit den Chemiluminescer-Partikeln assoziiert sind, reagieren, wobei die gebildete metastabile Verbindung unter Erzeugung eines Lichtblitzes wieder zerfällt. Da Singlet-Sauerstoff in wässrigen Lösungen nur kurze Zeit stabil ist, werden nur Chemiluminescer-Partikel, die z.B. durch Immunkomplexbildung in unmittelbarer Nähe zu den z.B. per Licht angeregten Sensitizer-Partikel liegen, angeregt, Licht abzustrahlen. Durch entsprechende Fluoreszenzfarbstoffe in den Chemiluminescer-Partikeln kann die Wellenlänge des zu messenden abgestrahlten Lichts verändert werden. Eine detaillierte Beschreibung des LOCl™-Verfahrens kann beispielsweise EP-A2-0 515 194 entnommen werden. Unter dem Begriff "Sensitizer-Partikel" sind insbesondere Mikropartikel zu verstehen, die mit einem oder mehreren Farbstoffen markiert sind, die unter Bestrahlung mit Licht Singlet-Sauerstoff generieren. Unter dem Begriff "Chemiluminscer-Partikel" sind insbesondere Mikropartikel zu verstehen, die Farbstoffe enthalten, die mit Singlet-Sauerstoff unter Lichtabstrahlung reagieren.

Bei den erfindungsgemäßen Verfahren kann es sich bei R1 und R2, bei R1 und R3, bei R1, R2 und R3 oder bei R2 und R3 um den gleichen Bindungspartner handeln, dies gilt insbesondere dann, wenn der nachzuweisende Analyt zumindest zwei gleiche Bindungsstellen aufweist. So haben z.B. viele bakterielle Antigene oder viele Tumormarker repetitive Epitope, so daß mehrere Exemplare eines monoklonalen Antikörpers an diese Antigene zu binden vermögen. Bei einem erfindungsgemäßen Testverfahren zum Nachweis solcher Antigene könnte folglich ein solcher monoklonaler Antikörper sowohl als Bindungspartner "R1 und R2", "R1 und R3", "R2 und R3" oder "R1, R2 und R3" eingesetzt werden. Bei einer besonders bevorzugten Ausführungsform handelt es sich bei R2 und R3 um den gleichen Bindungspartner, wobei R2 bevorzugterweise mit einer suspendierbaren Festphase assoziiert sein sollte.

Bei den erfindungsgemäßen Verfahren kann es sich ferner bei L1 und L2 um das gleiche Label handeln, z.B. Chemiluminescer-Partikel. So könnte in einem solchen Falle das Meßsignal der Chemiluminscer-Partikel als Label L1 turbidimetrisch oder nephelometrisch bestimmt werden und das Meßsignal der Chemiluminscer-Partikel als Label L2 plus L1 mit Hilfe eines Luminometers. Bei einer anderen Ausführungsform könnte das Meßsignal der Chemiluminscer-Partikel als Label L1 und das Meßsignal der Chemiluminscer-Partikel als Label L2 plus L1 jeweils zu verschiedenen Zeitpunkten mit Hilfe eines Luminometers bestimmt werden.

Bei einem besonders bevorzugten erfindungsgemäßen Verfahren handelt es sich bei der Festphase um eine suspendierbare Festphase, bevorzugt um Mikropartikel wie z.B. Latexpärtikel oder Magnetpartiket.

Die suspendierbare Festphase, insbesondere wenn es sich hierbei um Mikropartikel handelt, kann auch als Label fungieren. Eine besonders bevorzugte Festphase sind z.B. Latexpartikel, Magnetpartikel oder Sensitizer-Partikel.

Ganz bevorzugt ist auch ein erfindungsgemäßen Verfahren bei dem die Bindungspartner R2 mit einer suspendierbaren Festphase assoziiert sind, insbesondere wenn sie kovalent an diese gebunden sind. Bei der suspendierbaren Festphase handelt es sich hierbei insbesondere um Mikropartikel, besonders bevorzugt um Mikropartikel, die das Label L1 darstellen, oder ganz besonders bevorzugt um Chemiluminescer-Partikel.

Ein anderes bevorzugtes erfindungsgemäßes Verfahren ist dadurch gekennzeichnet, daß infolge der Sandwich-Bildung Komponenten eines signalbildenenden Systems, welches Label L1 und/oder Label L2 miteinschließt, in einen Abstand zueinander gebracht werden, der eine Wechselwirkung, insbesondere einen Energietransfer, zwischen diesen Komponenten erlaubt und das Ausmaß an Wechselwirkung gemessen wird, z.B. das weiter oben und in den Beispielen beschriebene Verfahren auf Basis der LOCl™-Technologie.

Bei einem ganz besonders bevorzugten erfindungsgemäßen Verfahren umfaßt das signalbildende System an Mikropartikel assoziierte Photosensitizer und an Mikropartikel assoziierte chemolumineszierende Substanzen.

Ferner umfaßt die Erfindung die Verwendung von einem an eine Festphase assoziierten Analyt A-spezifischen Bindungspartner R1, einem Analyt A-spezifischen Bindungspartner R2, der mit einem Label L1 assoziiert ist, und einem Analyt A-spezifischen Bindungspartner R3, der mit einem Label L2 assoziiert ist, zum Nachweis, zur Vermeidung und/oder zur Verminderung des Hook-Effektes in einem Verfahren, insbesondere in einem homogenen Sandwich-Test, zum quantitativen oder qualitativen Nachweis eines Analyten A in einer Probe. Die Absättigung der Analyt-A-Bindungsstellen der im Inkubationsansatz befindlichen Bindungspartner R2 erfolgt hierbei im Vergleich zur Absättigung der Analyt-A-Bindungsstellen der im Inkubationsansatz befindlichen Bindungspartner R3 bei einer höheren Analyt A-Konzentration und/oder zu einem späteren Inkubationszeitpunkt und das von L1 abhängige Meßsignal wird von dem von L2 oder von L1 plus L2 abhängigen Meßsignal entweder zeitlich voneinander getrennt oder mit Hilfe eines anderen Meßverfahrens bestimmt.

Auch erfaßt wird von der Erfindung die Verwendung von einem an eine Festphase assoziierten Analyt A-spezifischen Bindungspartner R1, einem Analyt A-spezifischen Bindungspartner R2, der mit einem Label L1 assoziiert ist, einem Analyt A-spezifischen Bindungspartner R3, der mit einem Mitglied X eines spezifischen Bindungspaars assoziiert ist, und einem Label L2, welches mit dem zu X korrespondierenden Bindungspaarmitglied Y eines spezifischen Bindungspaars assoziiert ist, zum Nachweis, zur Vermeidung und/oder zur Verminderung des Hook-Effektes in einem Verfahren, insbesondere in einem homogenen Sandwich-Test, zum quantitativen oder qualitativen Nachweis eines Analyten A in einer Probe. Die Absättigung der Analyt-A-Bindungsstellen der im Inkubationsansatz befindlichen Bindungspartner R2 erfolgt im Vergleich zur Absättigung der Analyt-A-Bindungsstellen der im Inkubationsansatz befindlichen Bindungspartner R3 bei einer höheren Analyt A-Konzentration und/oder zu einem späteren lnkubationszeitpunkt.

Ein anderer Bestandteil der Erfindung ist ein Testkit für einen heterogenen oder homogenen Sandwich-Test zum quantitativen oder qualitativen Nachweis eines Analyten A in einer Probe, der dadurch gekennzeichnet ist, daß er einen an eine Festphase assoziierten Analyt A-spezifischen Bindungspartner R1, einen Analyt A-spezifischen Bindungspartner R2, der mit einem Label L1 assoziiert ist, und einen Analyt A-spezifischen Bindungspartner R3, der mit einem Label L2 assoziiert ist, bevorzugt in getrennten Behältern enthält und wobei in dem Sandwich-Test die Absättigung der Analyt-A-Bindungsstellen der im Inkubationsansatz befindlichen Bindungspartner R2 im Vergleich zur Absättigung der Analyt-A-Bindungsstellen der im Inkubationsansatz befindlichen Bindungspartner R3 bei einer höheren Analyt A-Konzentration und/oder zu einem späteren Inkubationszeitpunkt erfolgt.

Ein anderer erfindungsgemäßer Testkit für einen heterogenen oder homogenen Sandwich-Test zum quantitativen oder qualitativen Nachweis eines Analyten A in einer Probe ist dadurch gekennzeichnet, daß er einen an eine Festphase assoziierten Analyt A-spezifischen Bindungspartner R1, einen Analyt A-spezifischen Bindungspartner R2, der mit einem Label L1 assoziiert ist, einen Analyt A-spezifischen Bindungspartner R3, der mit einem Mitglied X eines spezifischen Bindungspaars assoziiert ist, und ein Label L2, welches mit dem zu X korrespondierenden Bindungspaarmitglied Y eines spezifischen Bindungspaars assoziiert ist, bevorzugt in getrennten Behältern enthält und wobei in dem Sandwich-Test die Absättigung der Analyt-A-Bindungsstellen der im Inkubationsansatz befindlichen Bindungspartner R2 im Vergleich zur Absättigung der Analyt-A-Bindungsstellen der im Inkubationsansatz befindlichen Bindungspartner R3 bei einer höheren Analyt A-Konzentration und/oder zu einem späteren Inkubationszeitpunkt erfolgt. Besonders bevorzugt ist ein solcher Testkit, bei dem sich der Analyt A-spezifische Bindungspartner R2, der mit einem Label L1 assoziiert ist, und der Analyt A-spezifische Bindungspartner R3, der mit einem Mitglied X eines spezifischen Bindungspaars assoziiert ist, zusammen in einem Behälter befinden.

Ferner sind Testkits Bestandteil der Erfindung, die die zur Durchführung des erfindungsgemäßen Verfahrens benötigten Komponenten enthalten.

Die erfindungsgemäßen Testkits können auch eine Packungsbeilage, Verdünnungspuffer, Standards, Kontrollen, Systemreagenzien und/oder weitere für die Testdurchführung benötigte Komponenten enthalten. Besonders bevorzugte erfindungsgemäße Testkits enthalten eine Packungsbeilage, in der das erfindungsgemäße Verfahren beschrieben ist.

**Fig. 1** zeigt die schematische Darstellung eines bevorzugten erfindungsgemäßen Testverfahrens ("F" = Festphase). In einem ersten Schritt werden Festphase-R1, Analyt ("A"; sofem in der Probe vorhanden), R2-L1 und R3-L2 (oder R3-X) miteinander vermischt und dieser Inkubationsansatz bis zum Zeitpunkt T1 inkubiert.

Zum Zeitpunkt T1 wird das Meßsignal des im Bindungskomplex Festphase-R1-Analyt-R2-L1 enthaltenen Labels L1 bestimmt. Danach wird Y-L2 (nur bei R3-X) hinzugegeben und der Inkubationsansatz bis zum Zeitpunkt T2 inkubiert. Danach wird das Meßsignal des im Bindungskomplex Festphase-R1-Analyt-R3-X-Y-L2 befindlichen Labels L2 bestimmt. Sind L1 und L2 die gleichen Label wird bevorzugterweise zum Zeitpunkt T2 das Meßsignal beider in den Bindungskomplexen befindlichen Label L1 und L2 bestimmt. Anstatt der Messung des Meßsignals der gebundenen Label kann auch jeweils oder alternativ das Meßsignal des ungebunden Labelanteils, d.h. der nicht im Bindungskomplex befindlichen Label, im Inkubationsansatz gemessen werden.

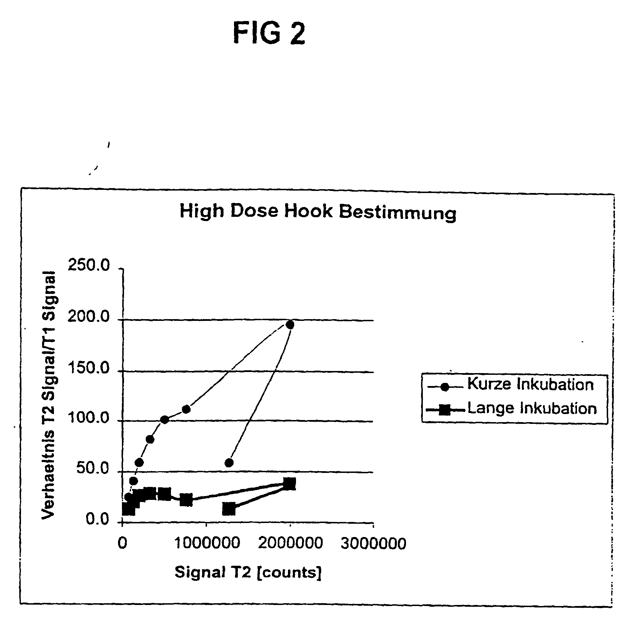

In **Fig. 2** ist die Bestimmung von High-Dose-Hook-Proben dargestellt, wobei die Signalhöhe zum Zeitpunkt T2 im Verhältnis zum "T2-Signal/T1 Signal-Wert" aufgetragen wird.

Die im folgenden beschriebenen Beispiele dienen zur exemplarischen Beleuchtung einzelner Aspekte dieser Erfindung und sind nicht als Einschränkung zu verstehen.

### BEISPIELE:

Verwendete-Abkürzungen:
- ADx: Aminodextran
- Biotin-X-NHS: Sulfosuccinimidyl-6-(biotinamido)-hexanoat
- BSA: Bovine Serum Albumin
- C-bead-ADx: Aminodextran beschichtete Chemiluminescer-Partikel
- C-bead-ADx-DxAl: Aminodextran und Dextranaldehyde beschichtete Chemiluminescer-Partikel
- CMO: Carboxymethyl oxime oder Carboxymethoxylamin hemihydrochlorid
- DMAP: 4-Dimethylamino-pyridin
- DMF: Dimethylformamid
- DMSO: Dimethylsulfoxid
- DxAI: Dextranaldehyd
- EDAC: 1-Ethyl-3-(Dimethylpropyl)carbodiimid
- MES: 2-(N-Morpholino)ethansulfonsäure
- MOPS: 3-N-(morpholino)propansulfonsäure
- NaBH₃CN: Natriumcyanoborhydrid
- NaHCO₃: Natriumhydrogencarbonat
- NaOH: Natriumhydroxid
- NH₂: Aminogruppe
- Sulfo-SMCC: Sulfosuccinimidyl 4-(maleimidomethyl)cyclohexane-1-carboxylate
- SC-ADx: Single coated Aminodextran
- S-bead-ADx: Aminodextran beschichtete Sensitizer-Partikel
- S-bead-DxAl: Dextranaldehyde beschichtete Sensitizer-Partikel
- S-bead-DxAl-SAV: Streptavidin beschichtet auf S-bead-DxAl
- STUT: O-(N succinimidyl)-N,N,N'-tetramethyluronium tetrafluoroborat
- TAPS: 3-[N-tris-(hydroxymethyl)methylamino]propansulfonsäure Natrium Salz
- TAR: Thioxen, Anthracen,Rubren
- Tris: Tris(hydroxymethyl)aminomethan
- Zn(BF₄)₂: Zinkfluoroborat

### Beispiel 1: Einfärbung der Latex-Partikel

### 1) Chemiluminescer-Partikel

Latex-Partikel (Seradyn, Katalog Nr.: 83000550100890; Durchmesser von ca. 0,2 µm) werden in einer Mischung aus Ethylenglycol, 2-Ethoxyethanol, und NaOH (65,4%, 32,2% und 2,3%) suspendiert und 40 Minuten bei 95°C ± 3°C gerührt. In einer 2-Ethoxyethanol-Lösung werden Thioxen (203 mg/g Partikel), 1-Chloro-9, 10-bis(phenylethylnyl)anthracen (16 mg/g Partikel) und Rubren (27 mg/g Partikel) bei 95°C ± 3°C 30 Minuten inkubiert. Beide Ansätze werden vereinigt und weitere 20 Minuten unter Rühren inkubiert. Nach diesen 20 Minuten wird die Partikel-Suspension auf 40 °C ± 10°C heruntergekühlt. Die gefärbten Partikel werden durch einen 43 Micron Mesh Polyesterfilter gegeben und gewaschen (Microgen Lab System). Zuerst werden die Partikel mit einem Lösungsmittelgemisch aus Ethylenglycol und 2-Ethoxyethanol (70% und 30%, 500 ml pro Gramm Partikel) und nachfolgend mit 10%igen Ethanol (pH 10 - 11, 400 ml pro Gramm Partikel) gewaschen.

### 2) Sensitizer-Partikel

Latex-Partikel (Seradyn, Katalog-Nr. 83000550100890; Durchmesser von ca. 0,2 µm) werden in einem Lösungsmittelgemisch aus 2-Ethoxyethanol, Ethylenglycol und NaOH (65,4%, 32,2% und 2,3%) suspendiert und 40 Minuten bei 95°C ± 3°C gerührt. Der Sensitizer-Farbstoff (t-Butyl Silicon Phthalocyanine) wird in Benzylalkohol gelöst (92 g Farbstoff/5ml/g Partikel) und 30 Minuten bei 95°C ± 3°C erhitzt. Nach Abschluß der beiden Inkubationszeiten werden bei Ansätze vereinigt und weitere 20 Minuten gerührt. Die Partikel-Suspension wird auf 40°C ± 10°C abgekühlt und durch einen 43 Micron Mesh Polyester Filter filtriert und anschließend gewaschen. Zuerst werden die Partikel mit Ethylalkohol (700 ml pro Gramm Partikel) und nachfolgend mit 10%igen Ethanol (pH 10 - 91, 600 ml pro Gramm Partikel) gewaschen.

### Beispiel 2: Herstellung von Aminodextran (ADx)

Mehrer Methoden zur Herstellung von Aminodextran sind bekannt. Eine Methode soll hier vorgestellt werden. Hydroxypropyldextran (1 NH₂/7 Glucose) wird hergestellt, in dem Dextran T-500 (Pharmacia, Uppsala, Schweden; 50 g) in 150 mL Wasser in einem Glaskolben mit Rührer und einem Tropfkolben aufgelöst wird. 18,8 g Zn(BF₄)₂ werden zu der Lösung gegeben und mit einem Wasserbad auf eine Temperatur von 87 °C gebracht. Epichlorohydrin (350 mL) wird innerhalb von 30 Minuten tropfenweise unter Rühren zu der Lösung hinzugegeben. Das Gemisch wird weitere 4 Stunden bei 85 °C bis 95 °C gerührt, dann auf Raumtemperatur abgekühlt. Das entstandene Chlorodextran wird praezipitiert, in dem die Lösung in 3 L Methanol unter Rühren gegeben, danach gefiltert und über Nacht in einem Vakuumofen getrocknet wird.

Das Chlorodextran wird in 200 mL Wasser gelöst und zu 2 L 36% Ammoniak gegeben. Die Lösung wird 4 Tage bei Raumtemperatur gerührt und dann auf 190 mL in einem Rotationsverdampfer eingeengt. Das Konzentrat wird in zwei gleiche Teile aufgeteilt und jeweils langsam in 2 L Methanol gegeben. Der Niederschlag wird abfiltriert und in einem Vakuumofen getrocknet.

Der getrocknete Niederschlag wird in 50 mM MOPS, pH 7,2 aufgelöst (12.5 mg/mL) Die Lösung wird 8 Stunden bei Raumtemperatur gerührt, abgekühlt (4 - 8°C) und für 45 Minuten bei 15000 Umdrehungen pro Minute in einer Sorvall RC-5B Zentrifuge zentrifugiert. Zu 10 mL des Überstandes werden 23,1 g Sulfo-SMCC in 1 mL Wasser gegeben. Die Mischung wir 1 Stunde inkubiert und ohne weitere Vorbehandlung zum Beschichten der gefärbten Chemiluminescer-Partikel eingesetzt (s. Beispiel 4).

### Beispiel 3: Herstellung von Dextranaldehyd (DxAl)

Dextranaldehyd wird hergestellt, in dem 400 g von Dextran T-500 (Phamacia, Uppsala, Schweden) in 1,5 L Wasser in einem 4 Literkolben bei 70 °C gerührt werden. Die Lösung wird filtriert und unter Argon eine Zn(BF₄)₂ Lösung (400 mL, 25 wt% in Wasser, pH 1,8) hinzugegeben. Allyl 2,3-epoxypropylaether wird portionsweise (3 x 500 mL, 8-10 mL/min) bei einer Temperatur von 70 °C hinzugeben. Für weitere 12-13 Stunden wird die Lösung bei 80 °C unter Argon gerührt. Danach wird das Reaktionsgemisch abgekühlt und in 6 L Wasser gegeben. Das verdünnte Gemisch wird ultrafiltriert mittels eines Microgen tangential flow diafiltration Systems und auf 1,0 - 1,5 L eingeengt.

Das Allyloxydextran wird dann unter, Rühren ozonyliert. Das Ozon wird mit einem Ozonator generiert und unter Druck (9.0 psi) mit einer Flussgeschwindigkeit von 2 Litern pro Minute durch die Allyloxydextran-Lösung geblubbert. 10 mL Heptanol wird als Antischaummittel hinzugefügt. Nach ca. 10 Stunden wird die Lösung auf 10 °C heruntergekühlt. 50 mL Dimethylsulfid werden unter Argon hinzugefügt. Nach 10 Stunden ständigem Rühren wird das Dextranaldehyd durch eine Microgen Ultrafiltration aufgereinigt.

### Beispiel 4: Herstellung von Aminodextran-beschichteten TAR-Partikeln (C-bead-ADx)

1 mL (22 mg/mL) der gefärbten Chemiluminescer-Partikel (Beispiel 1 Absatz 1) werden mit 1 mL einer Aminodextran-Lösung (20 mg/mL, MW 500K, s. Beispiel 2) in 0,05 MES, pH 6,0 in der Gegenwart von 3.8 mg/mL EDAC gemischt. Nach einer Inkubationszeit von 16 Stunden bei Raumtemperatur ("RT") im Dunkeln, werden die Partikel mit 2 mL 0,05 M MES, danach mit 6 ml 0,05 M MES (1 M NaCl, pH 6,0) gewaschen. Die Partikel werden in 1 mL 0,05 M MES, pH 6,0, aufgenommen (22 mg/mL SC-ADx-Partikel). Die Partikel werden durch Zentrifugation (Sorval RC-5 B Plus oder Eppendorf 5415C Zentrifuge) und Resuspendierung mittels Beschallung (Btanson Sonifier-450) gewaschen.

### Beispiel 5: Beschichtung der C-bead-ADx mit Dextranaldehyd

1 mL einer Dextranaldehyd-Lösung (20 mg/mL, s. Beispiel 3, MW 500K) und 1 mL einer 22 mg/mL C-bead-ADx Lösung (0,05 M MES, pH 6) werden zusammen mit 2 mg/mL NaBH₃CN inkubiert. Nach 20 Stunden Inkubation bei 37°C im Dunkeln werden die Partikel zweimal mit 5 mL MES Puffer gewaschen. Die Partikel werden dann in 0,5 mL 0,05 M MES, 0,4% Tween 20, pH 6, suspendiert (40 mg/mL C-bead-ADx-DxA1).

### Beispiel 6: Beschichten der C-bead-ADx-DxAl mit Anti-Human lgG Antikörpern

Antikörper beschichte Partikel werden hergestellt in dem gleiche Volumina einer Antikörper-Lösung (20 mg/mL anti-human.lgG Antikörper in 0,05M MES, pH 5,0) und einer Partikel-Suspension (40 mg/mL C-bead-ADx-DxAl in 0,05 M MES, 0,4% Tween-20, pH 6) in der Gegenwart von 0,5 mg/mL NaBH₃CN gemischt werden. Nach 16 Stunden Inkubation bei Raumtemperatur werden die verbliebenen Aldehydgruppen mit 0,08 M CMO (Carboxymethyl oxime oder Carboxymethoxylamine) für 90 Minuten bei 37 °C umgesetzt. Die Partikel werden dann 3 - 4 mal mit einem geeigneten Puffer gewaschen (Tris Puffer, 1 % BSA, 0.1 % Zwittergent 3-14). Die Konzentration der Partikel ist nach der letzten Resuspendierung 1 mg/mL.

### Beispiel 7: Herstellung von Dextranaldehyd beschichteten Sensitizer-Partikeln (S- bead-DxAl)

Die Sensitizer-Partikel aus Beispiel 1, Absatz 2 werden zu 20 mg/mL in Wasser verdünnt. Danach auf eine Konzentration von 18 mg/mL mit 300 mM TAPS Puffer, pH 9,0 eingestellt. Hydrazin, (36,3 µL/g Partikel), STUT (0,456 g/g Partikel) und DMAP (23,2 mg/g Partikel) werden hinzugefügt. STUT (frisch in DMF, 10%) wird in 4 Portionen alle 15 Minuten hinzugegeben. DMAP (frisch in DMF, 10%) wird nach der ersten STUT Zugabe dazugegeben. Der pH-Wert wird nach jeder STUT Zugabe auf pH 9,0 nachgestellt. Nach einer Stunde Rühren bei Raumtemperatur werden die Partikel mit dem 10-fachen Volumen TAPS Puffer, pH 9,0, gewaschen (Microgen System).

Die Partikel werden mit 1 mM TAPS Puffer aufgenommen (20 mg/mL) und langsam zu 40 mg/mL Dextranaldehyd in Acetatpuffer, pH 5,0, unter kräftigem Rühren gegeben. Nach 30 Minuten wird die Reaktionstemperatur auf 50 °C eingestellt und 18 weitere Stunden unter Rühren inkubiert. Die Partikel werden dann mit dem 25-fachen Volumen mit Wasser gewaschen (Microgen System)

### Beispiel 8: Herstellung von Streptavidin beschichteten Sensitizer-Partikeln (S-bead-DxAt-SAv)

Die Dextranaldehyd beschichteten Partikel (S-bead-DxAl, 100 mg/mL) werden zu einer Streptavidin-Lösung (30 mg/mL 10 mM Phosphatpuffer, pH 7) gegeben. Die Lösung wird eine Stunde bei 37 °C gerührt. Danach wird Natriumcyanoborhydrid unter Rühren hinzugeben (NaBH₃CN in Wasser, 50 mg/mL, 2% des Gesamtvolumens) und der Gesamtansatz weitere 40 - 72 Stunden bei 37 °C gerührt. Danach wird 1 M Carboxymethoxylamin (CMO) in Acetatpuffer, pH 5, hinzugegeben (1.5 % des Gesamtansatzes) und zwei weitere Stunden inkubiert. Danach werden die Partikel mit dem 25-fachen Volumen eines proteinfreien Puffers (0,1 M Tris, 0,3 M NaCl, 25 mM EDTA, pH 8,2) gewaschen.

### Beispiel 9: Herstellung von Rubella-Antigen beschichteten Sensitizer-Partikeln

Zur Herstellung von Rubella-Antigen beschichteten Sensitizer-Partikeln werden die Dextranaldehyd beschichteten Partikel (S-bead-DxAl) im ersten Schritt mit L-Lysin und im zweiten Schritt mit Succinic Anhydrid umgesetzt. An die Carboxyl-Gruppen wird dann das Rubella-Antigen gekoppelt. Zu 10 mL S-bead-DxAl Partikeln (100 mg/ml in 50 mM MES, 0,2% Tween 20, pH 6,0) werden 10 mL einer Lysin-Lösung (40 mg/mL in MES, pH 6,0) gegeben. 200 µL einer 10%igen Tween 20 Lösung werden hinzugefügt. 2 mL einer frisch zubereiteten Natrimcyanoborhydrid-Lösung (25 mg/mL in H₂O) werden hinzugegeben. Der Ansatz wird 4 Stunden bei 37 °C inkubiert. Danach werden erneut 2 mL einer frisch zubereiteten Natriumcyanoborhydrid-Lösung (25 mg/mL in H₂O) hinzugegeben. Der Reaktionsansatz wird für weitere 16 Stunden bei 37 °C auf dem Rollmixer inkubiert. Danach werden 10 mL Borat Puffer (200 mM, pH 9,0) hinzugegeben, 30 Minuten zentrifugiert (16 000 rpm, 10°C) und der Überstand verworfen. Das Pellet wird mit 15 mL Borat Puffer aufgenommen, und resuspendiert. Der Ansatz wird dann mit einem Sonifier 250. in einem Wasserbad sonifiziert (20 Pulse, Output 5, DC 50 %).

10 mL (500 mg) der S-bead-DxAl-Lysin Partikel werden mit 34,5 mL Borat Puffer gemischt. Zu dem Ansatz werden gegeben: 0,5 mL einer 10%igen Tween 20 Lösung und 1 mL einer Succinic Anhydrid Lösung (10 mg in 100 mL DMSO). Der Ansatz wird 2 Stunden bei Raumtemperatur gerührt. Es wird nochmals 1 mL einer Succinic Anhydrid Lösung (10 mg in 100 mL DMSO) hinzugegeben und 16 Stunden bei Raumtemperatur unter Rühren inkubiert. Es werden 10 mL MES Puffer (50 mM MES, pH 5,0) hinzugegen, zenrifugiert (30 Minuten, 16 000 rpm, 10 °C). Der Überstand wird verworfen und das Pellet mit 40 mL resuspendiert. Der Waschvorgang wird zwei- bis dreimal wiederholt. Zum Abschluss wird das Pellet in ca. 8 mL MES Puffer (50 mM, 0,1% Tween 20, pH 5,0) resuspendiert und mit dem Sonifier 250 homogenisiert (30 Pulse, Output 5, DC 50%).

### Beispiel 10: Herstellung von biotinyliertem Rubella-Antigen

Zur Herstellung von biotinyliertem Rubella-Antigen werden 6,5 mL der Rubella-Antigen-Lösung (0,6 mg/mL Rubella-Antigen in 0,1 M NaHCO₃/0,25 % Tween) mit 650 µL Biotin-X-NHS der Firma Pierce (1,2 mg/mL in DMSO) unter Rühren gemischt. Nach 22 Stunden wird über eine Entsalzungssäule der Firma Pierce (Presto™) der Ansatz mit 0,05 M Phosphat, 0,15 M NaCl, 0,25 % Tween 20, pH 7,6 entsalzt.

### Beispiel 11: Durchführung eines erfindungsgemäßen Immunoassays ("High-Dose-Hook-Assay")

10 µL Probe werden mit 50 µL einer Partikel Suspension (C-bead-ADx-DexA1 beschichtet mit Anti-Human IgG Antikörpern, 50 µg/mL, Beispiel 6) und 50 µL einer Rubella-Antigen beschichteten Partikel-Suspension (S-bead-OxAl beschichtet mit Rubella-Antigen, 0,2 mg/mL; Beispiel 9) und 50 µL einer biotinyliertem Rubella-Antigen-Lösung (5 µg/mL) gemischt und 196 Sekunden (bzw. 359 Sekunden) bei 37 °C inkubiert. Nach diesen 196 Sekunden (359 Sekunden) wird das erste Signal (Zeitpunkt T1) aufgenommen. Nach weiteren 81 Sekunden (bzw. unmittelbar nach T1) werden zu dem Gemisch 50 µL der S-bead-DxAl-SAv-Suspension (0,2 mg/mL) und 75 µL Assaypuffer (0,1 M Tris Puffer, 0,3 M NaCl, 25 mM EDTA, 1 mg/mL BSA, pH 8,2) hinzugegeben und 264 Sekunden bei 37 °C inkubiert. Danach wird das zweite Signal (Zeitpunkt T2) aufgenommen.

### Beispiel 12: Durchführung eines Standardassays

10 µL Probe werden mit 50 µL Partikel-Suspension (C-bead-ADx-DxAI beschichtet mit Anti-Human IgG Antikörpern, 50 µg/mL, Beispiel 6) und 50 µL biotinyliertem Rubella-Antigen-Lösung (5 µg/mL) gemischt und 277 Sekunden bei 37 °C inkubiert. Danach werden zu dem Gemisch 100 µL der S-bead-DxAI-SAv-Suspension (0,1 mg/mL) und 25 µL Assaypuffer hinzugegeben und 264 Sekunden bei 37 °C inkubiert. Danach wird das Signal (Zeitpunkt T2) aufgenommen.

In Tabelle 1 sind die Ergebnisse des verschiedenen Immunoassayformate gegenüber gestellt:

| Probenverdünnung der High-Dose-Hook-Probe | "High-Dose-Hook-Assay" (kurzes T1) T1-Signal [counts] | "High-Dose-Hook-Assay" (langes T1) T1-Signal [counts] | "High-Dose-Hook-Assay" T2-Signal [counts] | Standardassay T2-Signal [counts] |
|---|---|---|---|---|
| Unverdünnt | 21495 | 96651 | 1260815 | 1477950 |
| 1:2,5 | 10202 | 52762 | 1986820 | 2314755 |
| 1:5 | 6785 | 34393 | 759392 | 1283560 |
| 1: 10 | 4901 | 18097 | 498505 | 718458 |
| 1:20 | 3964 | 11551 | 323291 | 456184 |
| 1:40 | 3273 | 7399 | 192937 | 251804 |
| 1:60 | 3114 | 6257 | 127737 | 164181 |
| 1:100 | 2894 | 5461 | 71684 | 97251 |

Die Bestimmung der High-Dose-Hook-Probe kann nun anhand der Signalhöhe zum Zeitpunkt T1 oder anhand der Signalhöhe zum Zeitpunkt T2 bezogen auf das Signalverhältnis T2 zu T1 durchgeführt werden. Abbildung 2 verdeutlicht diesen Zusammenhang. Bei höfiertitrigen Proben wird auch das Signal zum Zeitpunkt T1 solange ansteigen bis auch hier der High-Dose-Hook-Bereich erreicht ist. Somit wird der Wert für T2 zu T1 wie auch der Wert für die Signalhöhe zum Zeitpunkt T2 immer mehr abnehmen. Kurze Inkubationszeiten (T1) können vorteilhafter sein, um die High-Dose-Hook-Probe besser identifizieren zu können (siehe auch Abb. 2).

## Patentansprüche

1. Verfahren zum quantitativen oder qualitativen Nachweis eines Analyten A in einer Probe, wobei die Probe mit einem an eine Festphase assoziierten Analyt A-spezifischen Bindungspartner R1, einem Analyt A-spezifischen Bindüngspartner R2, der mit einem Label L1 assoziiert ist, und einem Analyt A-spezifischen Bindungspartner R3, der mit einem Label L2 assoziiert ist, inkubiert wird und wobei die Absättigung der Analyt-A-Bindungsstellen der im Inkubationsansatz befindlichen Bindungspartner R2 im Vergleich zur Absättigung der Analyt-A-Bindungsstellen der im Inkubationsansatz befindlichen Bindungspartner R3 bei einer höheren Analyt A-Konzentration und/oder zu einem späteren Inkubationszeitpunkt erfolgt, **dadurch gekennzeichnet, daß** das von L 1. abhängige Meßsignal von dem von L2 oder von L1 plus L2 abhängigen Meßsignal entweder zeitlich voneinander getrennt oder mit Hilfe eines anderen Meßverfahrens bestimmt wird.

2. Verfahren nach Anspruch 1 zum Nachweis, zur Vermeidung und/oder zur Verminderung des Hook-Effektes.

3. Verfahren nach Anspruch 1 oder 2, **dadurch gekennzeichnet, daß** man (i) die Probe mit einem an eine Festphase assoziierten Analyt A-spezifischen Bindungspartner R1, einem Analyt A-spezifischen Bindungspartner R2, der mit einem Label L1 assoziiert ist, und einem Analyt A-spezifischen Bindungspartner R3, der mit einem Mitglied X eines spezifischen Bindungspaars assoziiert ist, inkubiert; (ii) zu einem späteren Zeitpunkt Label L2, welches mit dem zu X korrespondierenden Bindungspaarmitglied Y eines spezifischen Bindungspaars assoziiert ist, dem Testansatz hinzufügt; und (iii) das Meßsignal mindestens an zwei Zeitpunkten T1 und T2 bestimmt wird, wobei der frühere Zeitpunkt T1 spätestens kurz nach Zugabe von Label L2, welches mit Bindungspaarmitglied Y assoziiert ist, und der spätere Zeitpunkt T2 nach Zugabe von Label L2, welches mit Bindungspaarmitglied Y assoziiert ist, liegt.

4. Verfahren nach Anspruch 1 oder 2, **dadurch gekennzeichnet, daß** man (i) die Probe mit einem an eine Festphase assoziierten Analyt A-spezifischen Bindungspartner R1, einem Analyt A-spezifischen Bindungspartner R2, der mit einem Label L1 assoziiert ist, und einem Analyt A-spezifischen Bindungspartner R3, der mit einem Mitglied X eines spezifischen Bindungspaars assoziiert ist, inkubiert; (ii) zu einem späteren Zeitpunkt Label L2, welches mit dem zu X korrespondierenden Bindungspaarmitglied Y eines spezifischen Bindungspaars assoziiert ist, dem Testansatz hinzufügt; und (iii) das Meßsignal von L1 und das Meßsignal von L2 mit Hilfe von unterschiedlichen Meßverfahren bestimmt.

5. Verfahren nach einem der Ansprüche 1-4, wobei es sich beim Verfahren um einen heterogenen oder um einen homogenen Sandwich-Test handelt.

6. Verfahren nach einem der Ansprüche 1-5, wobei es sich bei R1 und R2, bei R1 und R3, bei R1, R2 und R3 oder bei R2 und R3 um den gleichen Bindungspartner handelt.

7. Verfahren nach einem der Ansprüche 1-6, wobei es sich bei L1 und L2 um das gleiche Label handelt.

8. Verfahren nach einem der Ansprüche 1-7, wobei es sich bei der Festphase um eine suspendierbare Festphase, bevorzugt Mikropartikel, handelt.

9. Verfahren nach Anspruch 8, wobei die Mikropartikel als Label fungieren.

10. Verfahren nach einem der Ansprüche 1-9, wobei der Bindungspartner R2 mit einer suspendierbaren Festphase, bevorzugt Mikropartikel, assoziiert ist.

11. Verfahren nach Anspruch 10, wobei die Mikropartikel das Label L1 darstellen.

12. Verfahren nach einem der Ansprüche 1-11, wobei infolge der Sandwich-Bildung Komponenten eines signatbildenden Systems, welches Label L1 und/oder Label L2 miteinschließt, in einen Abstand zueinander gebracht werden, der eine Wechselwirkung, insbesondere einen Energietransfer, zwischen diesen Komponenten erlaubt und das Ausmaß an Wechselwirkung gemessen wird.

13. Verfahren nach Anspruch 12, wobei das signalbildende. System an Mikropartikel assoziierte Photosensitizer und an Mikropartikel assoziierte chemolumineszierende Substanzen umfaßt.

14. Verwendung von einem an eine Festphase assoziierten Analyt A-spezifischen Bindungspartner R1, einem Analyt A-spezifischen Bindungspartner R2, der mit einem. Label L1 assoziiert ist, und einem Analyt A-spezifischen Bindungspartner R3, der mit einem Label L2 assoziiert ist, zum Nachweis, zur Vermeidung und/oder zur Verminderung des Hook-Effektes in einem Verfahren, insbesondere in einem homogenen Sandwich-Test, zum quantitativen oder qualitativen Nachweis eines Analyten A in einer Probe, und wobei die Absättigung der Analyt-A-Bindungsstellen der im Inkubationsansatz befindlichen Bindungspärtner R2 im Vergleich zur Absättigung der Analyt-A-Bindungsstellen der im Inkubationsansatz befindlichen Bindungspartner R3 bei einer höheren Analyt A-Konzentration und/oder zu einem späteren inkubationszeitpunkt erfolgt, **dadurch gekennzeichnet, daß** das von L1 abhängige Meßsignal von dem von L2 oder von L1 plus L2 abhängigen Meßsignal entweder zeitlich voneinander getrennt oder mit Hilfe eines anderen Meßverfahrens bestimmt wird.

## Revendications

1. Procédé pour la détection quantitative et qualitative d'un analyte A dans un échantillon, l'échantillon étant incubé avec un ligand R1 spécifique à l'analyte A associé à une phase solide, un ligand R2 spécifique à l'analyte A, qui est associé à un marqueur L1, et un ligand R3 spécifique à l'analyte A, qui est associé à un marqueur L2, et la saturation des sites de liaison à l'analyte A du ligand R2 présent dans la préparation d'incubation se faisant à une concentration en analyte A plus élevée et/ou à un moment de l'incubation plus tardif par rapport à la saturation des sites de liaison à l'analyte A du ligand R3 présent dans la préparation d'incubation, **caractérisé en ce que** le signal de mesure dépendant de L1 est déterminé par rapport au signal de mesure dépendant de L2 ou de L1 plus L2 soit de manière séparée dans le temps, soit à l'aide d'une autre méthode de mesure.

2. Procédé selon la revendication 1 pour détecter, éviter ou réduire l'effet Hook.

3. Procédé selon la revendication 1 ou 2, **caractérisé en ce que** (i) l'échantillon est incubé avec un ligand R1 spécifique à l'analyte A associé à une phase solide, un ligand R2 spécifique à l'analyte A, qui est associé à un marqueur L1, et un ligand R3 spécifique à l'analyte A, qui est associé à membre X d'une paire de liaison spécifique ; (ii) le marqueur L2, qui est associé à un membre de paire de liaison Y correspondant à X d'une paire de liaison spécifique, est ajouté ultérieurement à la préparation d'essai ; et (iii) le signal de mesure est déterminé au moins en deux instants T1 et T2, l'instant le plus précoce T1 se situant au plus tard peu de temps après l'addition du marqueur L2 qui est associé au membre d'une paire de liaison Y, et l'instant le plus tardif T2 se situant après l'addition du marqueur L2 qui est associé au membre d'une paire de liaison Y.

4. Procédé selon la revendication 1 ou 2, **caractérisé en ce que** (i) l'échantillon est incubé avec un ligand R1 spécifique à l'analyte A associé à une phase solide, un ligand R2 spécifique à l'analyte A, qui est associé à un marqueur L1, et un ligand R3 spécifique à l'analyte A, qui est associé à membre X d'une paire de liaison spécifique ; (ii) le marqueur L2, qui est associé à un membre de paire de liaison Y correspondant à X d'une paire de liaison spécifique, est ajouté ultérieurement à la préparation d'essai ; et (iii) le signal de mesure de L1 et le signal de mesure de L2 sont déterminés à l'aide de méthodes de mesure différentes.

5. Procédé selon une des revendications 1 à 4, le procédé étant un test sandwich hétérogène ou homogène.

6. Procédé selon une des revendications 1 à 5, R1 et R2, R1 et R3, R1, R2 et R3 ou R2 et R3 étant le même ligand.

7. Procédé selon une des revendications 1 à 6, L1 et L2 étant le même marqueur.

8. Procédé selon une des revendications 1 à 7, la phase solide étant une phase solide pouvant être mise en suspension, de préférence des microparticules.

9. Procédé selon la revendication 8, les microparticules servant de marqueur.

10. Procédé selon une des revendications 1 à 9, le ligand R2 étant associé à un phase solide pouvant être mise en suspension, de préférence des microparticules.

11. Procédé selon la revendication 10, les microparticules représentant le marqueur L1.

12. Procédé selon une des revendications 1 à 11, dans lequel, en raison de la configuration en sandwich, les composants d'un système formant les signaux qui comprend le marqueur L1 et/ou le marqueur L2 sont mis à une distance les uns des autres qui permet une interaction, en particulier un transfert d'énergie, entre ces composants, et dans lequel on mesure l'étendue de l'interaction.

13. Procédé selon la revendication 12, dans lequel le système formant les signaux comprend des photosensibilisateurs associés aux microparticules et des substances chimioluminescentes associées aux microparticules.

14. Utilisation d'un ligand R1 spécifique à l'analyte A associé à une phase solide, d'un ligand R2 spécifique à l'analyte A, qui est associé à un marqueur L1, et d'un ligand R3 spécifique à l'analyte A, qui est associé à un marqueur L2, pour détecter, pour éviter et/ou pour réduire l'effet Hook dans un procédé, en particulier un test sandwich homogène, pour la détection quantitative ou qualitative d'un analyte A dans un échantillon, la saturation des sites de liaison à l'analyte A du ligand R2 présent dans la préparation d'incubation se faisant à une concentration en analyte A plus élevée et/ou à un moment de l'incubation plus tardif par rapport à la saturation des sites de liaison à l'analyte. A du ligand R3 présent dans la préparation d'incubation, **caractérisé en ce que** le signal de mesure dépendant de L1 est déterminé par rapport au signal de mesure dépendant de L2 ou de L1 plus L2 soit de manière séparée dans le temps, soit à l'aide d'une autre méthode de mesure.

## Claims

1. A method for quantitatively or qualitatively detecting an analyte A in a sample, with the sample being incubated with an analyte A-specific binding partner R1, which is associated with a solid phase, an analyte A-specific binding partner R2, which is associated with a label L1, and an analyte A-specific binding partner R3, which is associated with a label L2, and with the saturation of the analyte A-binding sites of the binding partners R2 which are present in the incubation mixture taking place at a higher analyte A concentration and/or at a later time in the incubation than does the saturation of the analyte A-binding site of the binding partners R3 which are present in the incubation mixture, which comprises determining the L1-dependent measurement signal either at a different time from the L2-dependent or L1 plus L2-dependent measurement signal or using a different measurement method.

2. The method as claimed in claim 1 for detecting, avoiding and/or decreasing the hook effect.

3. The method as claimed in claim 1 or 2, wherein (i) the sample is incubated with an analyte A-specific binding partner R1, which is associated with a solid phase, an analyte A-specific binding partner R2, which is associated with a label L1, and an analyte A-specific binding partner R3, which is associated with a member X of a specific binding pair; (ii) at a later time, label L2, which is associated with the binding pair member Y, corresponding to X, of a specific binding pair, is added to the test mixture; and (iii) the measurement signal is determined at at least two times T1 and T2, with the earlier time T1 being at the latest shortly after adding label L2, which is associated with binding pair member Y, and the later time T2 being, after adding label L2, which is associated with binding pair member Y.

4. The method as claimed in claim 1 or 2, wherein (i) the sample is incubated with an analyte A-specific binding partner R1, which is associated with a solid phase, an analyte A-specific binding partner R2, which is associated with a label L1, and an analyte A-specific binding partner R3, which is associated with a member X of a specific binding pair; (ii) at a later time, label L2, which is associated with the binding pair member Y, corresponding to X, of -a specific binding pair, is added to the test-mixture; and (iii) the measurement signal of L1 and the measurement signal of L2 are determined using different measurement methods.

5. The method as claimed in one of claims 1-4, wherein the method is a heterogeneous or a homogeneous sandwich test.

6. The method as claimed in one of claims 1-5, wherein R1 and R2, R1 and R3, R1, R2 and R3, or R2 and R3, are the same binding-partner.

7. The method as claimed in one of claims 1-6, wherein L1 and L2 are the same label.

8. The method as claimed in one of claims 1-7, wherein the solid phase is a suspendable solid phase, preferably microparticles.

9. The method as claimed in claim 8, wherein the microparticles function as a label.

10. The method as claimed in one of claims 1-9, wherein the binding partner R2 is associated with a suspendable solid phase, preferably microparticles.

11. The method as claimed in claim 10, wherein the microparticles constitute the label L1.

12. The method as claimed in one of claims 1-11, wherein, as a consequence of the formation of the sandwich, components of a signal-forming system, which includes label 1 and/or label 2, are brought to a distance from each other which permits an interaction, in particular an energy transfer, between these components, and the extent of the interaction is measured.

13. The method as claimed in claim 12, wherein the signal-forming system comprises photosensitizers which are associated with microparticles and chemoluminescent substances which are associated with microparticles.

14. The use of an analyte A-specific binding partner R1, which is associated with a solid phase, an analyte A-specific binding partner, which is associated with a label L1, and an analyte A-specific binding partner R3, which is associated with a label L2, for detecting, avoiding and/or decreasing the hook effect in a method, in particular in a homogeneous sandwich test, for quantitatively or qualitatively detecting an analyte A in a sample, and with the saturation of the analyte A-binding sites of the binding partners R2 which are present in the incubation mixture taking place at a higher analyte A concentration and/or at a later time in the incubation than does the saturation of the analyte A-binding sites of the binding partners R3 which are present in the incubation mixture, wherein the L1-dependent measurement signal is determined either at a different time from the L2-dependent or L1 plus L2-dependent measurement signal or using a different measurement method.
